# EUROPEAN PATENT SPECIFICATION

(11) **EP 4 469 003 B1**
(45) Date of publication and mention of the grant of the patent: **20.08.2025**
(21) Application number: 23705651.0
(22) Date of filing: 24.01.2023
(51) Int. Cl.: A61F 2/24

(54) **PROSTHESIS FOR HEART VALVE AND METHOD FOR ARRANGING THIS PROSTHESIS IN AN IMPLANTATION DEVICE**
PROTHESE FÜR HERZKLAPPE UND VERFAHREN ZUR ANORDNUNG DIESER PROTHESE IN EINER IMPLANTATIONSVORRICHTUNG
PROTHÈSE POUR VALVULE CARDIAQUE ET PROCÉDÉ D'AGENCEMENT DE CETTE PROTHÈSE DANS UN DISPOSITIF D'IMPLANTATION

(30) Priority: 25.01.2022 IT 202200001166
(43) Date of publication of application: 04.12.2024
(73) Proprietor: Innovheart S.r.l., 20123 Milano (MI) (IT)
(72) Inventor: RIGHINI, Giovanni, 1196 Gland (CH); PANE, Gianluca, 10015 Ivrea (TO) (IT); BRESCIANO, Enrico, 20153 Milano (MI) (IT)
(74) Representative: Provvisionato, Paolo
(86) International application number: PCT/IB2023/050578
(87) International publication number: WO 2023/144691

(56) References cited:
- WO-A1-2021/014400

## Description

### Field of the invention

The present invention relates to the field of prostheses for heart valves.

The invention has been developed with particular regard, though in a non-limiting manner, to a prosthesis for a heart valve which is intended to replace the physiological function of a poorly working heart valve with particular though non-limiting reference to an atrioventricular heart valve. This prosthesis for a heart valve has been developed specifically for use by means of transcatheter implantation procedures.

The invention further relates to a method for arranging such a prosthesis for a heart valve in an implantation device which can be used in a transcatheter implantation procedure.

### Technological background

Heart valves are complex and delicate members which manage the correct function of the human heart. The main objective thereof involves making the haematic flow inside the cardiac cavities unidirectional, being necessary both during the phase of filling the cavities, the diastolic phase, and during the phase of discharging the blood, the systolic phase.

In order to optimize the efficiency of the pumping action of the blood, the heart is structured in two different compartments, the right and left compartments, respectively, each of which is in turn subdivided into two chambers, the atrium and ventricle, respectively. The right compartment of the heart, which is composed of the right atrium and ventricle, brings back the blood from the peripheral circulation and directs it towards pulmonary circulation for the oxygenation thereof. The left compartment, which is similarly subdivided into the left atrium and ventricle, supplies peripheral circulation, bringing back the oxygenated blood from the pulmonary circulation and pumping it towards the systemic circulation.

In order to make the haematic flow unidirectional inside the heart, a valve is positioned at the outlet of each chamber. The valves positioned at the outlet of the atria are called the atrioventricular valves because they connect the atrial chamber to the ventricular chamber of each side of the heart. In the right side of the heart, this valve is also called the tricuspid valve, in the left side it is usually referred to as the mitral valve. Finally, the valve which is positioned at the outlet of the right ventricle is called the pulmonary valve while the valve at the outlet of the left ventricle is called the aortic valve.

The pathologies which alter the function of a heart valve are among the most serious in the cardiovascular field. Among them, insufficiency of the mitral valve, or the inability thereof to completely close, is a valve pathology which is very debilitating because it reduces the efficiency of the pumping action of the left side of the heart, which is responsible for the blood circulation of the entire body.

In the current state of the art, the standard therapy for treating severe valve malfunctions is to replace the valve with an implantable prosthesis. In other cases, mainly in the case of mitral valve dysfunction, a valve repair is carried out. In both cases, an open-heart surgical procedure is carried out, which affords direct access to the malfunctioning valve. This procedure requires the temporary arrest of the heart and the generation, by means of suitable pumps and oxygen exchangers, of an artificial extra-corporeal blood circulation. Notwithstanding the refinement of the techniques for managing the cardiac arrest and the improvement of the extra-corporeal circulation systems, the open-heart therapy involves risks as a result of the invasive nature thereof and the duration of the procedure. In fact, implantable prostheses, both the ones for repair and the ones for replacement, which are usually used in conventional surgery typically require a long operation for being fixed at the implant site by means of specific suturing techniques. In some cases, it is not even possible any surgical therapy as a result of the general conditions of the patient, for example, as a result of the advanced age of the patient or the presence of concomitant pathologies.

In order to overcome these limitations, interventional procedures with low invasiveness, called transcatheter procedures, have been recently developed. To this end, radially collapsible prostheses which are able to self-secure at the implant site are used. The prostheses can be implanted by means of catheters which can navigate inside the vascular system and release the heart prosthesis by reaching the implantation site from a remote access created, for example, in a peripheral vessel, such as a vein or a femoral artery. The valve dysfunction can thereby be corrected with the heart still beating and with limited use of surgical practice. At present, the transcatheter techniques are in current clinical use only for the treatment of the aortic valve.

The situation is different with regard to the treatment of the dysfunctions of the atrioventricular valves, in particular for treating mitral insufficiency. The complex anatomical configuration of the valve and the structures which surround it, the variability of the pathologies, which are also very different from each other and which directly or indirectly affect the valve, make it extremely difficult to comply with the requirements for a safe and effective implantation in the mitral valve via the transcatheter route.

In recent years, there have been developed transcatheter prostheses for atrioventricular valves which can be implanted in accordance with different accesses and procedures. A first implantation methodology which is particularly indicated for the mitral valve provides for creating access to the native valve to be replaced through the apex of the left ventricle, which methodology has already been developed by the same Applicant (WO 2014/080338 and WO 2014/080339). The procedure provides for a thoracic incision in order to expose the apex of the left ventricle. Via the apex, a direct access to the ventricular chamber is surgically obtained, sometimes by means of temporary positioning, that is to say, in a manner limited to the duration of the procedure, of an apical port. Via the apical access, from time to time the catheters necessary for carrying out the implantation procedure are subsequently inserted.

Another methodology which has also been developed by the same Applicant (WO 2021/014400) instead provides for transseptal access. The term "transseptal access" is intended to be understood to be the access to the atrioventricular valve to be replaced which, starting from a peripheral femoral vein, ascends the inferior vena cava up to the right atrium. In the case of the mitral valve, it is also necessary to reach the left atrium via an opening which is created, with interventional methods, in the septum between the two atria. The left atrium affords antegrade access to the native mitral valve. In this manner, damage to, that is to say the perforation of, the left ventricle associated with the transapical procedure, which affords access from the ventricular side, that is to say retrograde, to the mitral valve, is prevented.

The valve prostheses implanted via the transcatheter route have been specifically studied to be able to assume a configuration with minimal radial dimension, in order thereby to be able to be inserted in a catheter for navigating inside the cardiovascular system of the patient. The patent application WO 2015/118464 by the same Applicant describes, for example, a transcatheter prosthesis which is particularly suitable for the purpose. The prosthesis has a support and interface structure with the native valve and a group of flexible prosthetic leaflets which are fixed to the interior thereof. The prosthesis structure particularly comprises a valve-operated central body, a containment portion which is formed by two or more arcuate segments and connecting blocks which are mounted at the end of connecting arms which are fixedly joined to the structure of the central body in order to provide mechanical continuity between the central body and the arcuate segments. The arms, similarly to the entire structure of the central body, are made from a material with super-elastic properties so that they can be temporarily deformed into an insertion configuration which can navigate inside the human cardiovascular system, for example, the femoral vein or the inferior vena cava, when the prosthesis is mounted at the distal end of a catheter, as set out in WO 2021/014400. When the catheter which conveys the prosthesis reaches the implantation site, or the native valve to be treated, the arms take up by spring back the configuration required for implanting the prosthesis. Still with reference to the release system of the transcatheter system described in the patent application WO 2021/014400, the solution adopted to maintain the connecting arms in the deformed configuration involves also inserting them inside a distal capsule which already receives the compressed central body. Retrieving the orientation of the connecting blocks is brought about by means of a partial withdrawal of the capsule of the catheter which allows both the connecting blocks and the connecting arms to be exposed and released.

This known solution requires that the distal capsule of the catheter must be sufficiently long to cover the deformed connecting arms and the connecting blocks. Since the distal capsule is the most rigid portion of the entire catheter, the greater is its length the less is the ability of the catheter itself to navigate, because it is less suitable for overcoming the tortuous anatomical features present in order to reach the implantation site from the peripheral access. Furthermore, because the dimensions of the capsule are determined by the diameter of the collapsed prosthesis therein, it is over-dimensioned with respect to the connecting blocks; consequently, inside the distal capsule the connecting blocks are not completely aligned with the axis of the catheter and, therefore, with the advance direction. This misalignment increases the friction between the connecting blocks and the guide wires which slide therein, increasing the resistance to the advance movement of the catheter and increasing the risk of release of particulates from the blocks themselves or from the guide wires above which the blocks slide.

### Statement of invention

One of the objects of the invention is to solve the problems of the prior art. In particular, it is intended to provide a prosthesis for a heart valve which is collapsible and which is also safe and stable during the release procedure from a suitable implantation catheter. Another object is to allow easy and controlled navigation of the implantation catheter, conveying the collapsed prosthesis, in order to overcome in a controlled manner the tortuous anatomical features which are encountered by the catheter itself during its advance from the peripheral vascular access to the native valve to be treated. Another object is to provide a device which is economical, simple, reliable to use and safe.

In order to achieve these objects and other objects, the application relates to a prosthesis according to the appended claims 1 to 9 and a method for arranging the prosthesis in an implantation device for a transcatheter procedure according to claim 10.

There is particularly described a prosthesis for a transcatheter procedure in which each connecting block between the central body of the prosthesis and the containment portion is provided with a mutual coupling mechanism suitable for selectively maintaining the blocks themselves, together with the connecting arms to which they are fixed, in a compact configuration. In the compact configuration, the connecting blocks are preferably arranged so as to have an overall radial dimension less than the overall radial dimension of the central body inside the distal capsule. Preferably, in the compact configuration the connecting blocks have a substantially coaxial orientation with respect to the implantation catheter. In this manner, it is ensured that the connecting blocks cannot obstruct or interfere in any manner with the advance of the catheter in the circulatory system of the patient. Furthermore, because this mutual coupling mechanism maintains the compact configuration stable until the intentional release of the mechanism brought about by the implanting person, it is possible to provide a distal capsule of the implantation catheter with a length approximately equal to the length of the central body of the collapsed prosthesis, that is to say, significantly shorter than in the prior art. In this manner, the implantation catheter is made more flexible and therefore more suitable for overcoming the tortuous anatomical features which are encountered when reaching the implantation site of the prosthesis.

### Brief description of the drawings

Additional features and advantages will be appreciated from the following detailed description of a preferred embodiment of the invention with reference to the appended drawings which are provided purely by way of non-limiting example and in which:
- Figure 1 is an exploded view which schematically shows a prosthesis for a heart valve according to the invention, in an expanded implantation configuration according to a first embodiment,
- Figure 2 shows the prosthesis for a heart valve of Figure 1 with only the central body inside a distal capsule and the connecting blocks which are provided with a mutual coupling mechanism in a compact configuration and, with broken lines, in the configuration ready for implantation,
- Figures 3a and 3b show the two connecting blocks which are equipped with a mutual coupling mechanism according to a first embodiment, respectively,
- Figure 4 shows two connecting blocks which are similar to those depicted in Figures 3a and 3b and which are connected to each other in order to bring about the compact configuration,
- Figures 5a and 5b show the two connecting blocks which are equipped with a mutual coupling mechanism according to a second embodiment, respectively,
- Figure 6 shows two connecting blocks which are similar to those depicted in Figures 5a and 5b and which are connected to each other in order to bring about the compact configuration,
- Figure 7 shows one of the two connecting blocks equipped with a symmetrical mutual coupling mechanism according to a third embodiment,
- Figure 8 shows one of the two connecting blocks equipped with a symmetrical mutual coupling mechanism according to a fourth embodiment,
- Figure 9 shows two connecting blocks, similar to the one depicted in Figure 8, in a state connected to each other in order to bring about the compact configuration.

### Detailed description

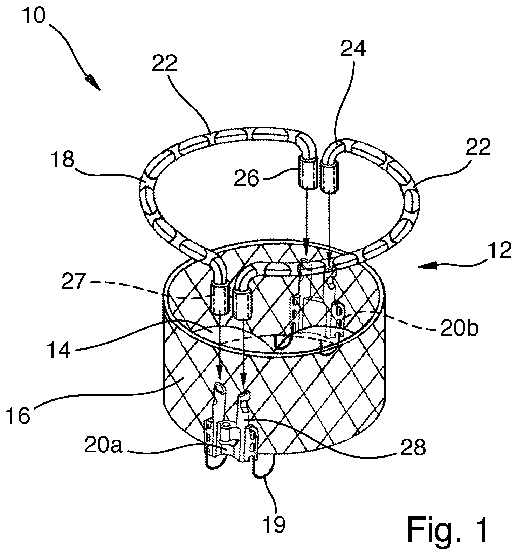

Now with reference to the drawings, Figure 1 shows an implantable prosthesis 10 which is used to replace the functionality of an atrioventricular valve.

The prosthesis 10 comprises a prosthetic structure 12 for supporting and interfacing with the native valve and a group of flexible prosthetic leaflets 14 which are fixed therein. The prosthetic structure 12 particularly comprises a central body 16, a containment portion 18 and connecting blocks 20a, 20b for mechanically connecting the containment portion 18 to the central body 16 via a series of connecting arms 19 which are fixedly joined to the central body.

The prosthetic structure 12, as well as each of the elements thereof, is configured so as to be collapsible without any negative effects on the safety and the functionality of the heart prosthesis. Therefore, it is possible to temporarily reduce the radial encumbrance of the prosthesis 10 in order to allow the introduction thereof inside the heart cavities through small vascular accesses which are compatible with surgical methods involving minimal invasiveness, by means of transcatheter procedures for the positioning and the implantation of cardiac prostheses and in particular with transcatheter procedures with transfemoral access, which is typical for the tricuspid heart valve, and transseptal access. In other words, it is possible to insert the cardiac prosthesis inside a catheter with a low radial profile which is capable of conveying the prosthesis from peripheral vascular accesses with minimal invasiveness as far as the interior of the heart cavity, near the site of implantation, and to carry out at that location the deployment and implantation thereof, functionally replacing the native valve.

In greater detail, the central body 16 is the portion of the prosthetic structure which delimits the conduit for the passage of the blood through the device. The flexible prosthetic leaflets 14 which make the blood flow unidirectional inside the conduit are fixed inside the central body 16, as known, for example, from the Italian Patent No. 20140204 from the same Applicant.

The central body 16 is an elastic structure which is radially collapsible and which tends, as a result of springback, to expand to a diameter even greater than the diameter of the containment portion 18.

The central body 16 is provided with arms 19, on which the connecting blocks 20a, 20b are provided. The connecting blocks are fixed to the arms 19 in a stable and permanent manner. The fastening is preferably mechanical, preferably reversible, but a different fastening method, for example by welding, is not excluded. It is also not to be excluded that the connecting blocks and arms are made in one piece.

The containment portion 18 is the portion of the prosthetic structure 10 which opposes and limits the free expansion of the central body 16, preventing it from exceeding the maximum diameter which is compatible with the preservation of the coaptation among the prosthetic leaflets 14. The containment portion 18 has a substantially annular geometry and is longitudinally non-extensible, that is to say, it does not significantly modify the peripheral development thereof even when the central body 16 expands therein, by applying a radial force outwardly.

The containment portion 18 is preferably subdivided into two sub-components 22 which are separated from each other and which are substantially arcuate. Each sub-component 22 can be selectively coupled with the connecting blocks 20a, 20b, to which it is then fixedly joined in the final implantation configuration.

Each end 24 of each sub-component 22 is equipped with a coupling portion 26. The connecting blocks 20a, 20b are equipped with pins 28 which can be received in axial holes 27 present in the coupling portions 26. It is evident that the pin/hole connection mechanism could instead comprise a pin at the end of the sub-components 22 and a hole, which is preferably cylindrical, in the connecting blocks 20a, 20b. More generally, the pin/hole connection has a merely exemplary purpose without any limiting intention concerning the general nature of the solution.

During use, the leaflets of the native valve remain trapped inside the connection between the central body 16 and the containment portion 18.

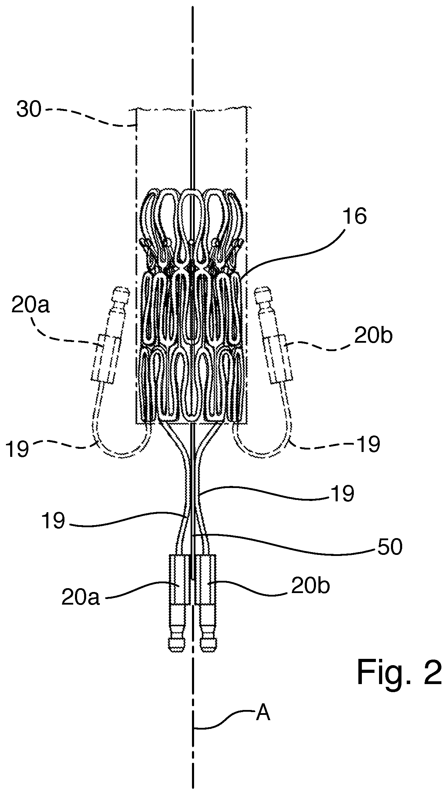

Now with reference to Figure 2, in order to allow the implantation with a transcatheter procedure the arms 19 are flexible. In particular, they can be elastically deformed so as to move from an expanded configuration (broken lines in Figure 2) which corresponds to the use configuration of the prosthesis, to a compact configuration (depicted with solid lines) which allows the advance of the central body of the prosthesis which is mounted at the distal end of the implantation catheter, in the cardiovascular system of the patient. In the expanded configuration, each arm 19 is curved and brings about an increase in the overall radial encumbrance of the prosthesis. In the compact configuration, however, each arm 19 is aligned approximately parallel with an axis A of the prosthesis. The arms 19 are preferably made from a material with super-elastic properties, for example Nitinol, so that they take up, in the absence of constraints, the expanded configuration by springback.

Figure 2 shows a prosthetic structure 12 which is made compact inside a distal capsule 30 of the implantation catheter which covers only the central body 16. The distal capsule 30 according to this embodiment therefore has a shorter length with respect to the distal capsule of the prior art. The connecting arms 19 and the connecting blocks 20a, 20b are maintained in the compact configuration which is aligned with the axis of the catheter by means of a mutual coupling mechanism which is formed directly on the connecting blocks themselves. This mutual coupling mechanism allows selective maintenance of a configuration of the connecting blocks parallel with the axis of the catheter and therefore in the optimum configuration having minimal resistance to the advance of the catheter. This solution also allows the length of the capsule to be reduced to the minimal dimension. Since the end capsule is the most rigid portion with respect to the deflection of the catheter and in particular with respect to the immediately proximal portion, this results in a catheter which is generally more flexible with respect to the ones known previously and therefore characterized by a greater level of navigability in particularly tortuous anatomies.

A suitable mechanism which can be remotely operated maintains the connecting blocks joined together and allows the intentional release thereof so as to again take up the expanded configuration, which is necessary in order to carry out the final step of implantation of the prosthesis.

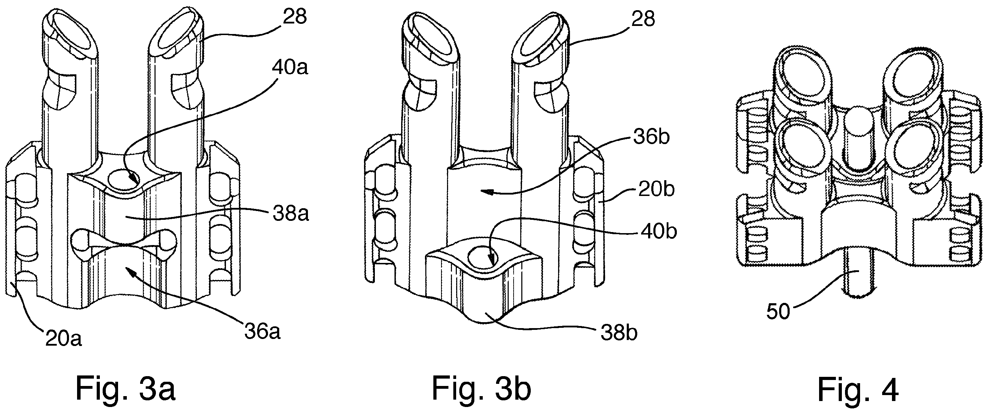

Some examples of a mutual coupling mechanism with which the connecting blocks are provided are described below with reference to Figures 3 to 8. This mutual coupling mechanism allows the connecting blocks 20a, 20b to be fixedly joined to each other. In this manner, the blocks themselves and the arms 19 can be maintained in the compact configuration which is suitable for allowing the navigation of the catheter in the cardiovascular system of the patient in order to reach the implantation site.

In the first embodiment, which is shown in detail in Figures 3a, 3b and 4, the two connecting blocks 20a, 20b are formed differently from each other. The connecting block 20a is provided, on the face 36a facing towards the connecting block 20b in the compact configuration described above, with a projection 38a. Similarly, the connecting block 20b is provided with a projection 38b on the face 36b facing towards the connecting block 20a in the compact configuration. Each of the blocks is provided with a respective through-hole 40a, 40b which are located more specifically in the respective projections 38a, 38b which constitute coupling mechanisms. The two projections and the holes are formed in such a manner that the two connecting blocks, when beside each other, have the respective projections adjacent to each other and the respective holes coaxial with each other. In this manner, it is possible to insert in both the same connecting element, by way of non-limiting example a guide wire 50 which thereby retains the blocks side by side. Since each block is subjected to an elastic force by the respective arm 19, which is equal and opposite, the blocks therefore remain in the compact configuration of Figure 2b. In this position, the holes have axes which are substantially coincident with the axis A of the prosthesis. In Figure 4, the two connecting blocks 20a and 20b are shown joined together by means of a guide wire 50. The guide wire 50 is coaxial with the prosthesis itself and extends through the entire catheter. With the guide wire 50 in position, the connecting blocks are kept joined together. By removing the guide wire 50, the connecting blocks are instead released, allowing the arms to take up the original configuration again. Naturally, instead of a guide wire it is possible to use, as the connecting element, a wire of a suitable diameter, which is preferably made of metal or another suitable material, or another elongate element.

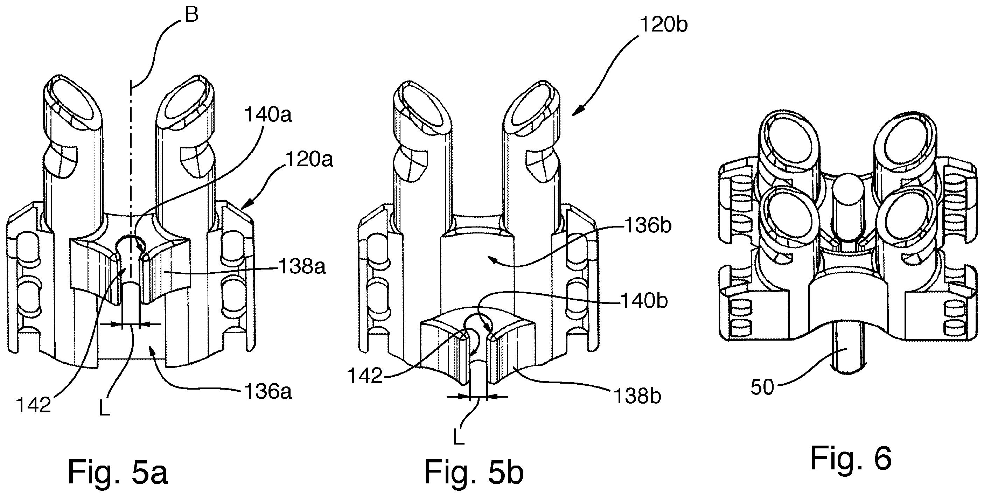

Now with reference to Figures 5a, 5b and 6, two connecting blocks 120a, 120b are formed in a generally equivalent manner to the connecting blocks 20a, 20b described above, with the single difference that they have open eyelets in place of the through-holes. The connecting block 120a is provided with a projection 138a on the face 136a which faces, in the compact configuration, towards the connecting block 120b. The connecting block 120b is provided with a projection 138b on the face 136b which faces towards the connecting block 120a in the compact configuration. The projections 138a, 138b are shaped so as to each have an open channel 140a, 140b for a guide wire 50; each channel defines a longitudinal direction B and is formed in such a manner that the guide wire 50 can be inserted into and removed from the channel only by sliding in the longitudinal direction B. The opening 142 of the channels 140a, 140b therefore has a width L less than the diameter of the guide wire. The two projections and the respective channels are formed in such a manner that the two projections are adjacent and the two mutually adjacent connecting blocks have the respective channels coaxial relative to each other. Therefore, they constitute a mutual coupling mechanism. In Figure 6, the two connecting blocks 120a, 120b are shown joined to each other by means of a wire 50.

This configuration with respect to the preceding configuration has the advantage of being able to be cleaned more easily in the haematic flow, preventing the accumulation of haematic or biological deposits in the through-hole. This configuration also has the additional advantage of allowing the release of the connecting blocks without having to necessarily completely remove the connecting element. In a non-limiting, exemplary manner, with reference to Figure 6, the wire 50 generally having a cross-section with a diameter greater than the dimension L, it can be profiled so as to have an intermediate portion with a cross-section with a diameter less than the width L of the opening 142. Initially, the two connecting blocks can be kept joined together by inserting the wire 50 in the channels 140a and 140b in the portion with a cross-section having a diameter greater than L. When wishing to release the connecting blocks in order to again take up the expanded configuration suitable for implantation, it is simply necessary to axially slide the wire 50, bringing the section with a diameter smaller than L into alignment with the openings 142, but without necessarily having to remove it completely from the implantation catheter. In this manner, it is possible to continue to use the wire 50 as a guide wire for the implantation catheter even after the release of the connecting blocks. In another variant, the same result can be achieved by constructing in at least one portion of the wire 50 an oblong or elliptical section or a section which is in any case characterized by two diameters, of which one is greater than the dimension L of the opening 142, while the second one is smaller than the dimension L of the opening 142. In this manner, the connecting blocks are mutually coupled when the wire 50 is orientated with the greater dimension aligned so as to interfere with the opening 142. The rotation of the wire 50, by bringing the smaller diameter of the section to face the opening 142, causes the release of the connecting blocks, allowing the arms to again take up the original configuration.

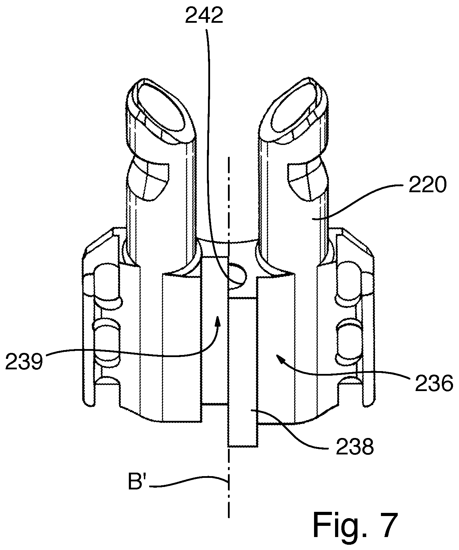

Figure 7 shows an additional variant, in which the two connecting blocks 220 of the same prosthesis are identical to each other. The connecting block 220 is provided with a projection 238 and a recess 239 on the face 236 which faces, in the compact configuration, towards the other connecting block 220. The projection 238 and the recess 239 are such that the projection of one block 220 is completely in the recess of the other block 220 which is beside it. The two adjacent blocks together form a hole for a guide wire 50, having an axis B'. The hole is defined by respective grooves 242 which are formed in the projections 238 and which are mutually adjacent.

When the guide wire 50 is inserted between the grooves 242, it prevents the blocks from becoming separated similarly to what is described for the coupling mechanisms of the preceding embodiments. However, the single groove 242 on the single block 220 in the absence of an additional adjacent block is not sufficient to retain the block 220 in the compact position. This configuration also, not having through-holes, allows the accumulation of deposits to be prevented.

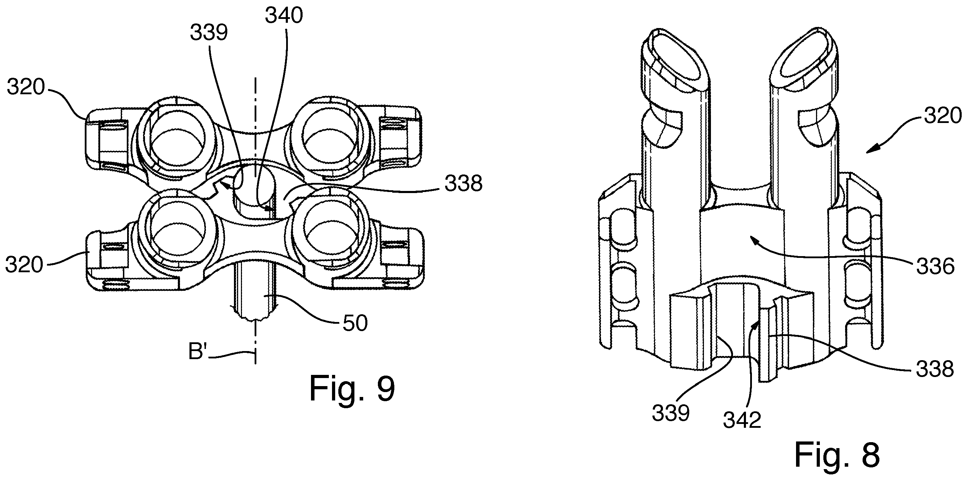

Figure 8 shows an additional variant of a block 320 which is similar to the preceding variant. In this case, the two connecting blocks of the same prosthesis are also mutually identical with a projection 338 and a recess 339 on the face 336 which faces, in the compact configuration, towards the other connecting block 320. Two adjacent blocks 320 together form a hole 340 for receiving a connecting element, such as a wire 50, the hole being defined by the respective grooves 342 which are formed on the projections 338, which are mutually adjacent. The difference with respect to the preceding embodiment is that the projection 338 and the recess 339 are formed in such a manner that, when the connecting element 50 is inserted in the hole 340, the profiles of the two blocks interfere mechanically with each other, preventing the separation between the two blocks and reducing the force to which the connecting element 50 is subjected. The interference between two blocks 320 can be seen in Figure 9 which shows two connecting blocks 320 connected to each other by a wire 50.

This configuration has, similarly to the one shown in Figure 6, the additional advantage of allowing the connecting blocks to be released without necessarily completely removing the wire which keeps them connected, which can continue to be used for other purposes, such as, for example, being the guide wire for the implantation catheter. In fact, it is simply necessary for the wire 50 to have a portion with such a reduced cross-section as to generate a play in the hole 340 which is sufficient to disengage the projections 338 from the grooves 342 and therefore to release the two connecting blocks from each other. Moving the wire 50 from a portion with a greater cross-section to a portion with a smaller cross-section brings about the result of releasing the two connecting blocks without necessarily having to remove the wire 50 from the implantation catheter. In a similar manner, a wire 50 which is profiled with an oblong section characterized by a greater dimension and a smaller dimension also allows the connecting blocks to be kept connected when the greater dimension of the cross-section is orientated so as to keep the projections 338 pressed against the corresponding recesses 339. By rotating the wire 50, the two coupling profiles become detached and the two connecting blocks become separated.

There should not further be excluded any other configuration which allows a block to be coupled with a guide wire or other similar elongate element. Purely by way of example, it is possible to provide on the connecting blocks eyelets of various types which are also constructed with threads, for example, suture threads.

Prostheses having connecting blocks according to the various embodiments described and according to other variants which may be derived by analogy all require a similar method in order to be arranged in a device for implanting a prosthesis with a transcatheter procedure, for example, such as the one described in the patent application WO 2021/014400. The method requires crimping of the expansible central body, the arrangement of the arms in the compact configuration, with the connecting blocks adjacent to each other and mutual coupling of the connecting blocks by inserting therein a guide wire 50 in the suitable seats which are formed in different manners. When the prosthesis is at the implantation site and it is necessary to bring the connecting blocks into an expanded configuration, it is simply necessary to withdraw the guide wire 50 to a sufficient extent for it to become disengaged from the connecting blocks. As a result of the elasticity of the arms 19, the connecting blocks will be automatically brought into the expanded configuration, in which they may be connected to the sub-components 22 of the containment portion.

The above-described embodiments always make reference to the presence of two connecting blocks, equal to the number of sub-components 22 of the containment portion in the configuration of the prosthesis of Figure 1. Naturally, the prosthesis may also comprise a different number of sub-components 22 of the containment portion, and therefore of connecting blocks. The version described, with two sub-components 22, is the preferred one because it allows the use of two guide wires which, as described in the patent application WO 2021/014400, are easier to position correctly than a single guide wire which could remain entangled in the chordae tendineae. However, it should not be excluded that the containment portion may comprise a single sub-component and may therefore be formed in the manner of a cut ring. In this case, it is advantageous for the single connecting block to have a configuration similar to those of Figures 1 to 6, which as a result of the geometry thereof allow a longitudinal element to be retained in the respective hole 40, 140 even in the presence of a single connecting block. It will further be preferable to use a bar with a given terminal rigidity in place of the guide wire 50 in order to be able to maintain the connecting block in a position which is as coaxial as possible relative to the prosthesis.

There should also not be excluded the solution which has a third sub-component, notwithstanding the greater complexity required by the positioning operations, mainly for a prosthesis which is intended for replacing the tricuspid heart valve. If there are provided three or more sub-components and therefore three or more connecting blocks, it is simply necessary to modify the profile thereof in such a manner that they can be adjacent to each other in groups of three, maintaining a through-hole or in any case a seat for a guide wire in each connecting block, in such a manner that the presence of the elongate element which can slide in the seat thereof in each of the adjacent connecting blocks brings about the coupling between the connecting blocks.

Finally, the above-described embodiments can also be actuated without any connecting blocks which are separated with respect to the connecting arms which are fixedly joined to the central body. It is simply necessary for the profiles and the coupling mechanisms described above to be constructed directly on the structure of the connecting arms.

It should also be noted that in order to ensure that the native valve leaflets remain properly trapped between the central body 16 and the containment portion 18, it is also possible to provide a central body that expands to a predetermined maximum diameter, which allows for proper coaptation between the prosthetic leaflets, embraced by a containment portion that retains the leaflets. A prosthesis so configured may also include connection blocks between the central body and the sub-components of the containment portion similar to those described above.

Naturally, the principle of the invention remaining the same, the forms of embodiment and details of construction may be varied widely with respect to those described and illustrated without thereby departing from the scope of the invention.

## Claims

1. A prosthesis for a heart valve for implantation with a transcatheter procedure comprising an expansible central body (16) and a containment portion (18) having one or more sub-components (22), the central body being provided with at least one elastically flexible arm (19), ending in a connecting block (20a, 20b, 120a, 120b, 220, 320) for connecting each sub-component of the containment portion (18) to the central body (16), wherein the arms are formed so as to be able to take up an expanded configuration, into which each arm returns without any constraints, and a compact configuration suitable for allowing the positioning of the prosthesis with a transcatheter procedure, **characterised in that** each connecting block is provided with a coupling mechanism (40a, 40b, 140a, 140b, 242, 340) suitable for selectively maintaining the corresponding arm in the compact configuration.

2. A prosthesis for a heart valve according to claim 1, wherein each arm is curved in the expanded configuration and is aligned approximately along an axis (A) of the prosthesis in the compact configuration.

3. A prosthesis for a heart valve according to claim 1 or 2, wherein the coupling mechanism is suitable for coupling each connecting block (20a, 20b, 120a, 120b, 220, 320) with an elongate element (50), which can selectively slide coaxially relative to the prosthesis for a heart valve or which can be selectively rotated angularly.

4. A prosthesis for a heart valve according to claim 1, 2 or 3, wherein the containment portion comprises at least two sub-components (22) and wherein the coupling mechanism of each connecting block (20a, 20b, 120a, 120b, 220, 320) is a mutual coupling mechanism, suitable for maintaining each block coupled with at least one other connecting block.

5. A prosthesis for a heart valve according to claim 4, wherein the mutual coupling mechanism of each connecting block (20a, 20b, 120a, 120b, 220, 320) comprises a housing seat (40a, 40b, 140a, 140b, 242, 342) for a slidable elongate element (50) which is formed on each connecting block in such a manner that the presence of the slidable elongate element in the seat thereof in each of the connecting blocks brings about the coupling between connecting blocks and the arrangement of the arms (19) in the compact configuration.

6. A prosthesis for a heart valve according to any one of claims 4 to 5, wherein the connecting blocks (220, 320) are mutually identical.

7. A prosthesis for a heart valve according to claim 5, wherein the housing seat for a slidable elongate element (50) of each connecting block (20a, 20b) comprises a through-hole (40a, 40b).

8. A prosthesis for a heart valve according to claim 5, wherein the housing seat for a slidable elongate element (50) of each connecting block (120a, 120b) comprises a channel which defines a longitudinal direction (B) and which is formed in such a manner that the slidable elongate element can be inserted in the channel of each connecting block only by sliding in the longitudinal direction (B).

9. A prosthesis for a heart valve according to claim 5 or 6, wherein the respective housing seats of each connecting block (220, 320) each comprise a groove (242, 342), the grooves of the two connecting blocks being formed in such a manner that, when the connecting blocks are mutually adjacent, they form a channel for the slidable elongate element which can be inserted in the channel simply by sliding in a longitudinal direction (B) of the channel itself.

10. A method for arranging a prosthesis according to claim 1 in an implantation device for a transcatheter procedure, the method comprising the steps of:
- crimping the expansible central body (16),
- arranging the at least one elastically flexible arm (19) in the compact configuration,
- coupling each connecting block (20a, 20b, 120a, 120b, 220, 320) by means of the respective coupling mechanism in order to maintain the at least one elastically flexible arm (19) in the compact configuration.

## Patentansprüche

1. Prothese für eine Herzklappe zur Implantation mit einem Transkatheterverfahren, umfassend einen ausdehnbaren Zentralkörper (16) und einen Einschließungsabschnitt (18), der eine oder mehrere Teilkomponenten (22) aufweist, wobei der Zentralkörper mit mindestens einem elastisch biegsamen Arm (19) bereitgestellt ist, der in einem Verbindungsblock (20a, 20b, 120a, 120b, 220, 320) endet, um jede Teilkomponente des Einschließungsabschnitts (18) mit dem Zentralkörper (16) zu verbinden, wobei die Arme so ausgebildet sind, dass sie eine expandierte Konfiguration, in die jeder Arm ohne jegliche Beschränkungen zurückkehrt, und eine kompakte Konfiguration, die geeignet ist, das Positionieren der Prothese mit einem Transkatheterverfahren zu ermöglichen, einnehmen können, **dadurch gekennzeichnet, dass** jeder Verbindungsblock mit einem Kopplungsmechanismus (40a, 40b, 140a, 140b, 242, 340) bereitgestellt ist, der geeignet ist, den entsprechenden Arm in der kompakten Konfiguration selektiv zu halten.

2. Prothese für eine Herzklappe nach Anspruch 1, wobei jeder Arm in der expandierten Konfiguration gekrümmt ist und in der kompakten Konfiguration etwa entlang einer Achse (A) der Prothese ausgerichtet ist.

3. Prothese für eine Herzklappe nach Anspruch 1 oder 2, wobei der Kopplungsmechanismus geeignet ist, jeden Verbindungsblock (20a, 20b, 120a, 120b, 220, 320) mit einem länglichen Element (50) zu koppeln, das zu der Prothese für eine Herzklappe selektiv koaxial verschiebbar oder selektiv winklig drehbar ist.

4. Prothese für eine Herzklappe nach Anspruch 1, 2 oder 3, wobei der Einschließungsabschnitt mindestens zwei Teilkomponenten (22) umfasst und wobei der Kopplungsmechanismus jedes Verbindungsblocks (20a, 20b, 120a, 120b, 220, 320) ein gegenseitiger Kopplungsmechanismus ist, der geeignet ist, jeden Block mit mindestens einem anderen Verbindungsblock gekoppelt zu halten.

5. Prothese für eine Herzklappe nach Anspruch 4, wobei der gegenseitige Kopplungsmechanismus jedes Verbindungsblocks (20a, 20b, 120a, 120b, 220, 320) einen Aufnahmesitz (40a, 40b, 140a, 140b, 242, 342) für ein verschiebbares längliches Element (50) umfasst, das an jedem Verbindungsblock derartig ausgebildet ist, dass das Vorhandensein des verschiebbaren länglichen Elements in dem Sitz davon in jedem der Verbindungsblöcke das Koppeln zwischen Verbindungsblöcken und die Anordnung der Arme (19) in der kompakten Konfiguration bewirkt.

6. Prothese für eine Herzklappe nach einem der Ansprüche 4 bis 5, wobei die Verbindungsblöcke (220, 320) miteinander identisch sind.

7. Prothese für eine Herzklappe nach Anspruch **5,** wobei der Aufnahmesitz für ein verschiebbares längliches Element (50) jedes Verbindungsblocks (20a, 20b) ein Durchgangsloch (40a, 40b) umfasst.

8. Prothese für eine Herzklappe nach Anspruch **5,** wobei der Aufnahmesitz für ein verschiebbares längliches Element (50) jedes Verbindungsblocks (120a, 120b) einen Kanal umfasst, der eine Längsrichtung (B) definiert und derartig ausgebildet ist, dass das verschiebbare längliche Element in den Kanal jedes Verbindungsblocks nur durch Verschieben in der Längsrichtung (B) eingesetzt werden kann.

9. Prothese für eine Herzklappe nach Anspruch 5 oder 6, wobei die jeweiligen Aufnahmesitze jedes Verbindungsblocks (220, 320) jeweils eine Nut (242, 342) umfassen, wobei die Nuten der zwei Verbindungsblöcke derartig ausgebildet sind, dass sie, wenn die Verbindungsblöcke miteinander benachbart sind, einen Kanal für das verschiebbare längliche Element ausbilden, das in den Kanal einfach durch Verschieben in einer Längsrichtung (B) des Kanals selbst eingesetzt werden kann.

10. Verfahren zum Anordnen einer Prothese nach Anspruch 1 in einer Implantationsvorrichtung für ein Transkatheterverfahren, wobei das Verfahren die folgenden Schritte umfasst:
- Anpressen des ausdehnbaren Zentralkörpers (16),
- Anordnen des mindestens einen elastisch biegsamen Arms (19) in der kompakten Konfiguration,
- Koppeln jedes Verbindungsblocks (20a, 20b, 120a, 120b, 220, 320) mittels des jeweiligen Kopplungsmechanismus, um den mindestens einen elastisch biegsamen Arm (19) in der kompakten Konfiguration zu halten.

## Revendications

1. Prothèse pour une valvule cardiaque pour l'implantation avec une procédure par cathéter comprenant un corps central expansible (16) et une partie de confinement (18) ayant un ou plusieurs sous-composants (22), le corps central étant prévu avec au moins un bras élastiquement flexible (19), se terminant par un bloc de raccordement (20a, 20b, 120a, 120b, 220, 320) pour raccorder chaque sous-composant de la partie de confinement (18) au corps central (16), dans laquelle les bras sont formés afin de pouvoir prendre une configuration expansée, dans laquelle chaque bras revient en l'absence de contraintes, et une configuration compacte appropriée pour permettre le positionnement de la prothèse avec une procédure par cathéter, **caractérisée en ce que** chaque bloc de raccordement est prévu avec un mécanisme de couplage (40a, 40b, 140a, 140b, 242, 340) approprié pour maintenir sélectivement le bras correspondant dans la configuration compacte.

2. Prothèse pour une valvule cardiaque selon la revendication 1, dans laquelle chaque bras est courbé dans la configuration expansée et est aligné approximativement le long d'un axe (A) de la prothèse dans la configuration compacte.

3. Prothèse pour une valvule cardiaque selon la revendication 1 ou 2, dans laquelle le mécanisme de couplage est approprié pour coupler chaque bloc de raccordement (20a, 20b, 120a, 120b, 220, 320) avec un élément allongé (50), qui peut sélectivement coulisser de manière coaxiale par rapport à la prothèse pour une valvule cardiaque ou qui peut être sélectivement entraîné en rotation de manière angulaire.

4. Prothèse pour une valvule cardiaque selon la revendication 1, 2 ou 3, dans laquelle la partie de confinement comprend au moins deux sous-composants (22) et dans laquelle le mécanisme de couplage de chaque bloc de raccordement (20a, 20b, 120a, 120b, 220, 320) est un mécanisme de couplage mutuel, approprié pour maintenir chaque bloc couplé avec au moins un autre bloc de raccordement.

5. Prothèse pour une valvule cardiaque selon la revendication 4, dans laquelle le mécanisme de couplage mutuel de chaque bloc de raccordement (20a, 20b, 120a, 120b, 220, 320) comprend un siège de logement (40a, 40b, 140a, 140b, 242, 342) pour un élément allongé coulissant (50) qui est formé sur chaque bloc de raccordement de sorte que la présence de l'élément allongé coulissant dans son siège dans chacun des blocs de raccordement occasionne le couplage entre les blocs de raccordement et l'agencement des bras (19) dans la configuration compacte.

6. Prothèse pour une valvule cardiaque selon l'une quelconque des revendications 4 à 5, dans laquelle les blocs de raccordement (220, 320) sont mutuellement identiques.

7. Prothèse pour une valvule cardiaque selon la revendication 5, dans laquelle le siège de logement pour un élément allongé coulissant (50) de chaque bloc de raccordement (20a, 20b) comprend un trou débouchant (40a, 40b).

8. Prothèse pour une valvule cardiaque selon la revendication 5, dans laquelle le siège de logement pour un élément allongé coulissant (50) de chaque bloc de raccordement (120a, 120b) comprend un canal qui définit une direction longitudinale (B) et qui est formé de sorte que l'élément allongé coulissant peut être inséré dans le canal de chaque bloc de raccordement uniquement en coulissant dans la direction longitudinale (B).

9. Prothèse pour une valvule cardiaque selon la revendication 5 ou 6, dans laquelle les sièges de logement respectifs de chaque bloc de raccordement (220, 320) comprennent chacun une rainure (242, 342), les rainures des deux blocs de raccordement étant formées de sorte que, lorsque les blocs de raccordement sont mutuellement adjacents, ils forment un canal pour l'élément allongé coulissant qui peut être inséré dans le canal simplement en coulissant dans une direction longitudinale (B) du canal lui-même.

10. Procédé pour agencer une prothèse selon la revendication 1 dans un dispositif d'implantation pour une procédure par cathéter, le procédé comprenant les étapes consistant à :
sertir le corps central expansible (16),
agencer le au moins un bras élastiquement flexible (19) dans la configuration compacte,
coupler chaque bloc de raccordement (20a, 20b, 120a, 120b, 220, 320) au moyen du mécanisme de couplage respectif afin de maintenir le au moins un bras élastiquement flexible (19) dans la configuration compacte.
